# EUROPEAN PATENT APPLICATION

(11) **EP 4 725 902 A1**
(43) Date of publication of application: **15.04.2026**
(21) Application number: 24823089.8
(22) Date of filing: 12.04.2024
(51) Int. Cl.: C01B 32/40, B01J 23/755, C01B 3/16, C01B 3/26, C01B 3/32, C01B 3/36, C01B 32/05, C01B 32/15, C07B 61/00, C07C 1/04, C07C 1/12, C07C 9/04, C07C 9/08

(54) **METHOD FOR PRODUCING CARBON MONOXIDE OR SYNTHESIS GAS, AND METHOD FOR PRODUCING CHEMICAL PRODUCT, METHANE OR PROPANE**

(30) Priority: 12.06.2023 JP 2023096631; 27.12.2023 JP 2023221412; 13.01.2024 JP 2024003641; 24.02.2024 JP 2024026074
(71) Applicant: Ihara Co., Ltd., Toyokawa-City, Aichi 441-0105 (JP); Umeda, Yoshito, Nagoya-City, Aichi 460-0008 (JP)
(72) Inventor: IHARA, Ryoseki, Toyokawa-City, Aichi 441-0105 (JP); SUZUKI, Ryota, Toyokawa-City, Aichi 441-0105 (JP); UMEDA, Yoshito, Nagoya-City, Aichi 460-0008 (JP)
(74) Representative: Nederlandsch Octrooibureau
(86) International application number: PCT/JP2024/014891
(87) International publication number: WO 2024/257467

(57) **Abstract**

To provide a novel method of producing CO or synthesis gas with less concern about producing water, and by applying such novel method, a method in which hydrogen is not essentially outsourced for cyclic usage of CO₂ produced via an oxidation reaction of hydrocarbons. Provided is a method of producing carbon monoxide or synthesis gas, comprising the steps of proceeding the reactions represented by the formula (1) (CH₄ → C + 2H₂) and the formula (2) (C + CO₂ → 2CO) in a sequential order within the identical container substantially in the anhydrous condition, wherein the reactions are conducted using a structural catalyst having a catalytic action of methane pyrolysis reaction, and a method of producing methane comprising (S1) to produce CO₂ following the oxidation reaction of methane, (S2) to produce hydrogen by the reaction of the above formula (1), (S3) to produce CO by the reaction of the above formula (2), and (S4) to produce methane by the reaction of hydrogen and CO, wherein the (S2) and (S3) are conducted in a sequential order within the identical container containing a structural catalyst having a catalytic activity of methane pyrolysis reaction.

## Description

### [Technical Field]

The present invention relates to a novel synthetic method of carbon monoxide with less concern of producing water, a method of producing a chemical product by applying such method or by utilizing an apparatus capable of implementing such method, and a method of producing methane or propane by the cyclic usage of CO₂ produced by city gas oxidation etc.

### [Background Art]

Synthesis gas mainly composed of carbon monoxide and molecular hydrogen produced from natural gas modification becomes a raw material to make various synthetic compounds such as fuel, synthetic resins and pharmaceutical products.
A number of reactions are proposed to produce synthesis gas. Each reaction has a suitable use, depending on the H₂/CO ratio of synthesis gas produced.

| Name of reaction of synthesis gas production | Chemical Formula | reaction heat | H₂/CO ratio | Major applications |
|---|---|---|---|---|
| Steam Reforming (SRM) | CH₄+H₂O → CO+3H₂ | endoergic | H₂/CO =3 | - Synthetic fuel production combined with DRM |
| | | | | - Hydrogen production combined with aqueous gas shift reaction (CO+H₂O → CO₂+H₂) |
| Direct Contact Partial Oxidation method (D-CPOX method) | CH₄+1/2O₂ → CO+2H₂ | exoergic | H₂/CO ≃2 | - Synthetic fuel production by Fischer-Tropsch reaction |
| | | | | - Methanol synthesis |
| Partial Oxidation method (POX method) | CH₄+1/2O₂ → CO+2H₂ | exoergic | 2>H₂/ CO>1 | Acetic acid/methacrylic acid production using CO |
| Classical Dry Reforming (DRM) | CH₄+CO₂ → 2CO + 2H₂ | endoergic | H₂/CO =1 | Alcohol production by oxo synthesis |

It can be said that steam reforming method (SRM) is the most practical method for producing synthesis gas. On the other hand, this method has too high H₂/CO ratio of synthesis gas for the use in producing synthetic fuels or methanol. It is thus necessary to lower the H₂/CO ratio by the combination with the other method. The production apparatus also tends to become too large.

Direct Contact Partial OXidation method (D-CPOX method) and Partial OXidation method (POX method) are exoergic reactions, which are ideal methods for producing synthesis gas in terms of input energy, however, these methods have problems of the difficulty of controlling reactions, lower thermal efficiency, the precipitation of heavy hydrocarbons and carbon, and the difficulty of continuous operation.

Recently, a focus is made on methane dry reforming, which uses raw materials of methane and carbon dioxide that become a cause of greenhouse effects as an environmentally green reforming (non-Patent Literature 1 etc.).

In a classical dry reforming reaction (Formula (3)) where synthesis gas is produced at a high temperature from methane and carbon dioxide, the aggregation of catalyst particles on a surface of the support as well as carbon precipitation often result in a lower catalytic activity in the supported catalyst used. Therefore, it is known to be hard to maintain catalytic activity (Patent Literature 1, non-Patent Literature 2, non-Patent Literature 3). Furthermore, it is often the case that the reaction for producing chemical products using CO₂ as a raw material generates water as a side product together with a desired product. This is mainly caused by reverse water-gas shift reaction (Formula (4)) where a product hydrogen is oxidized into water.

CH₄ + CO₂ → 2CO + 2H₂ (3)

CO₂ + H₂ → CO + H₂O (4)

Co-produced water causes 1) the decrease in equilibrium conversion rate due to the progress of reverse reaction and 2) the loss of catalytic activity due to the reaction of catalyst and water (catalyst poisoning), which impedes the effective progress of the reaction (non-Patent Literature 4).

The synthesis of methanol or polycarbonate raw material is a typical example in which co-produced water causes a problem. Therefore, it is considered as one of the fundamental technical challenges in producing an useful chemical product from raw material CO₂ to develop a reaction system capable of effectively removing water co-produced from reaction system (non-Patent Literature 4).

By the way, methanation is expected to become a core technology of "next-generation heat energy industries" in "green growth strategy associated with the carbon neutrality by 2050" developed in June 2021, considered as an important area expected to grow, and the development of the fundamental techniques is ongoing.

Since 2018 in France and since 2021 in Japan, there are manufacturers that start the demonstration of the systems for the cyclic usage of CO₂ generated from plant sites which oxidize city gas by the following formula (a) via the conventional methanation reaction represented by the following formula (b) (non-Patent Literature 5, non-Patent Literature 6).

CH₄ + 2O₂ → CO₂ + 2H₂O (a)

4H₂ + CO₂ → CH₄ + 2H₂O (b)

As is obvious from the above formula (b), however, 4 Nm³ hydrogen is required in the conventional methanation reaction to produce 1 Nm³ methane and use it while achieving carbon neutrality (Patent Literature 2). This hydrogen should be produced on a plant site or outsourced.

In producing hydrogen on a plant site, it necessitates renewable energy apparatus such as solar power generation, water electrolysis hydrogen production apparatus, storage facilities and pipes (Patent Literature 3, Patent Literature 4, Patent Literature 5, Patent Literature 6, Patent Literature 7). In usual, the enormous amount of CO₂ is generated on plant site, so it is totally impossible for such apparatuses to cover hydrogen demand. As a consequence, one would produce and receive hydrogen produced offsite. In such case, it necessitates delivery vehicles, compressor, storage facilities and drivers on the supply side, storage facilities on the receiving side. These are all initial cost factors.

Consequently, the initial cost is reflected on the running cost. The anticipated CIF price for hydrogen itself is 30 yen in 2030 and 20 yen in 2050 per 1 Nm³ of hydrogen. Thus, in conventional methanation processes, the cost of using hydrogen (120 yen /Nm₃·CH₄ in 2030, 80 yen /Nm₃·CH₄ in 2050) will be added on top of the cost that has been incurred for the simple oxidation use of methane. Therefore, the high running cost of hydrogen remains a challenge even in the future (non-Patent Literature 7).

### [Prior Art Document]

### [Patent Literature]

[Patent Literature 1] Japanese Unexamined Patent Application Publication No. 2012-188321
[Patent Literature 2] Japanese Unexamined Patent Application Publication No. 2023-66417
[Patent Literature 3] Japanese Unexamined Patent Application Publication No. 2022-060640
[Patent Literature 4] National Publication of International Patent Application No. 2023-527415
[Patent Literature 5] Japanese Unexamined Patent Application Publication No. 2015-196619
[Patent Literature 6] Japanese Unexamined Patent Application Publication No. 2005-60137
[Patent Literature 7] International Publication No. WO2018/099709

### [Non-Patent Literature]

[non-Patent Literature 1] Ogo et al., "Catalyst for methane, carbon dioxide and hydrogen", Chemistry and Education Vol. 66, No. 2, 2018 (URL: https://www.jstage.jst.go.jp/article/kakyoshi/66/2/66_68/_pdf)
[Non-Patent Literature 2] Cabinet Office, "Comprehensive study on technical problem of preferable technique in energy/environmental field ", March 2018 (URL: https://www8.cao.go.jp/cstp/stmain/houkokusho.pdf)
[Non-Patent Literature 3] Fukuhara et al., "A system of Ni-based structural catalyst reaction with combined dry reforming field of CH4 and carbon capturing field", The Japan Petroleum Institute, 66th workshop program, May 2017 (URL: https://www.jstage.jst.go.jp/article/sekiyu/2017/0/2017_5/_pdf)
[Non-Patent Literature 4] Cabinet Office, "Bottleneck issue in CO2 utilization and trend of research and development", page 18, February 2018 (URL: https: //www8.cao.go.jp/cstp/stmain/20180221bottleneck.html)
[Non-Patent Literature 5] Takagi, "Trend of methanation techniques", The 1st public-private council for promotion of methanation, Document 8, June 2021 (URL: https://www.meti.go.jp/shingikai/energy_environment/methanation_suishin/pdf/001_08 _00.pdf)
[Non-Patent Literature 6] Denso's website, "Denso starts demonstrative experiment of CO2 circulation plant in Electrification Innovation Center of Anjo Plant", April 7, 2021 (URL: https://www.denso.com/jp/ja/news/newsroom/2021/20210407-01/)
[Non-Patent Literature 7] Agency for Natural Resources and Energy, "Basic Hydrogen Strategy", pages 12 to 13, June 2023 (URL: https://www.meti.go.jp/shingikai/enecho/shoene_shinene/suiso_seisaku/pdf/20230606_ 2.pdf#page=12)
[Non-Patent Literature 8] Agency for Natural Resources and Energy, "Achievement of the goal of hydrogen/fuel cell strategy roadmap", page 3, June 25, 2019 (URL: https://www.meti.go.jp/shingikai/energy_environment/suiso_nenryo/roadmap_hyoka_w g/pdf/001_03_00.pdf#page=3)
[Non-Patent Literature 9] Agency for Natural Resources and Energy, "Standard Heating Values and Carbon Emission Coefficients by Energy Source (Revised in FY2018)", page 75, (URL: https://www.enecho.meti.gojp/statistics/total_energy/pdf/stte_028.pdf#page=82)
[Non-Patent Literature 10] INPEX website (URL: https://www.inpex.co.jp/ir/unit.html)

### [Summary of Invention]

### [Problem to be solved by the invention]

Should a carbon-neutral methanation process be developed with less expensive raw materials without the use of hydrogen, it may lead to promoting the methanation process.

The present invention has been made in view of the aforementioned current situation and the objective of the present invention is to provide a novel method of producing CO or synthesis gas with less concern about producing water, and by applying such novel method, a method in which hydrogen is not essentially outsourced for cyclic usage of CO₂ produced via an oxidation reaction of hydrocarbons.

### [Means for solving the problem]

The present inventors have focused on the fact that the methane dry reforming reaction of formula (3) may be divided into formula (1) and formula (2):

CH₄ + CO₂ → 2CO + 2H₂ (3)

CH₄ → C + 2H₂ (1)

C + CO₂ ↔ 2CO (2)

(1) The formula (1) is methane pyrolysis. The present inventors have discovered that the reaction progresses in a higher conversion rate by subjecting nickel-based structural catalyst having an interlayer of copper to radiation heat (e.g. International Publication No. 2021/079660 pamphlet, International Publication No. 2023/013182 pamphlet).

The formula (2) is a reversible reaction, also commonly known as Boudouard reaction.

If the reactions of formulae (1) and (2) may be smoothly progressed to right sides, one can substantially push the methane dry reforming reaction of formula (3) forward while avoiding the reaction of formula (4) from pushing toward water production.

One aspect of the present invention to achieve the above objective is a method of producing carbon monoxide or carbon monoxide and molecular hydrogen, comprising the steps of proceeding the reactions represented by the following formulae (1) and (2) in a sequential order within the identical container substantially in the anhydrous condition, wherein the reaction of the following formula (1) is conducted using a structural catalyst having a catalytic activity of methane pyrolysis reaction:
According to this method (Sequential Dry Reforming of Methane (SDRM)), the reactions represented by the formulae (1) and (2) are conducted in a sequential order substantially in the anhydrous condition. Therefore, H₂ and CO may be separately produced without co-producing water from the reaction system due to reverse water-gas shift reaction etc. Since each reaction is conducted within the identical container, heat for proceeding one reaction can be effectively utilized for the other reaction. The identical catalyst can be used without exchange.

In the aforementioned method, it may be preferable that carbon dioxide (CO₂) is CO₂ stored by the carbon dioxide collection/storage (Carbon Capture Storage; CCS) technique. This is because there is a problem of exploring the use of large amount of CO₂ separated from CCS (non-Patent Literature 4), and this method is regarded as one of the promising use of high-concentration CO₂ obtained without separation or purification, and furthermore, the obtained CO may be the only industrially applicable compound capable of reducing CO₂ (non-Patent Literature 2, page 95).

In the aforementioned method, the aforementioned reaction of formula (2) may be conducted in the presence of carbon produced in said reaction of formula (1). This allows us to effectively consume and utilize produced carbon as a raw material of CO when it is not collected.

Another aspect of the present invention is a method of producing a synthesis gas or a chemical product using the same, comprising the step of mixing carbon monoxide and molecular hydrogen obtained by said method in a certain ratio by the molar flow rate adjustment. The method of producing a synthesis gas or a chemical product may further comprise the step of adding molecular hydrogen from renewable energy to adjust a H₂/CO ratio. This allows us to adjust the H₂/CO ratio to any level from around 1 to beyond 2 obtained by SDRM method.

Another aspect of the present invention is a reaction apparatus comprising: a reaction furnace containing a structural catalyst having a catalytic activity of methane pyrolysis reaction; and a CO detector on a flow path communicated with a gas outlet of said reaction furnace. Such configuration allows us to manufacture a small-size apparatus suitable for the implementation of SDRM method capable of producing carbon monoxide by use of carbon produced by the catalytic pyrolysis reaction of methane. Since a structural catalyst is used, unlike supported catalysts, there is less risk of reactor blockage due to the deposited carbon (such as filamentous carbon or carbon nanotubes). Accordingly, the temporary storage capacity of the produced carbon is less likely to decrease, thereby reducing the risk of an increase in the energy required to supply the reaction gases.

The reaction apparatus may preferably comprise a reaction furnace with a gas inlet and a gas outlet, and further comprise a three-way valve on an inlet side to switch a supply path to said gas inlet and a three-way valve on an outlet side to switch a discharging path from said gas outlet. Such configuration may reduce the number of pipes directly connected to the apparatus. The apparatus may be operated by alternatingly switching two statuses: the supply path of CO₂ and the discharging path of CO are automatically closed when said three-way valve on an inlet side is open to the supply path of CH₄ and said three-way valve on an outlet side is open to the discharging path of H₂; and the supply path of CH₄ and the discharging path of H₂ are automatically closed when said three-way valve on an inlet side is open to the supply path of CO₂ and said three-way valve on an outlet side is open to the discharging path of CO. Therefore, it becomes an apparatus further suitable for the implementation of SDRM method.

The reaction apparatus may preferably comprise an outlet valve to control the discharge of molecular hydrogen and/or CO from said reaction furnace, and a main valve capable of discharging a gas remained in said reaction furnace into the atmosphere or a main valve capable of releasing said remained gas into a flow path different from either hydrogen flow path or CO flow path. Such main valve allows us to improve the purity of produced gas to be controlled by the outlet valve downstream of the main valve through the purging operation with raw material gas at the main valve.

One aspect of the present invention to achieve the above objective is a method of producing methane or propane by cyclic usage of CO₂ produced following oxidation reaction of hydrocarbons, comprising: a step (1) of producing molecular hydrogen and/or CO₂ following oxidation reaction of hydrocarbons; a step (2) of producing C and molecular hydrogen by pyrolysis of hydrocarbons; a step (3) of producing CO by reacting CO₂ produced in the step (1) with carbon produced in the step (2); and a step (4) of producing methane or propane by reacting CO produced in the step (3) with molecular hydrogen produced in the step (1) or step (2), wherein the reactions of said step (2) and step (3) are conducted in a sequential order substantially in the anhydrous condition within the identical container containing a structural catalyst having a catalytic action of methane pyrolysis reaction. Such method makes it unnecessary to supply renewable energy consistently in the methanation/propanation process and makes it sufficient to use only hydrocarbons as a raw material in producing methane or propane and using it while achieving carbon neutrality. Furthermore, hydrocarbon cost may be reduced by selling a part of carbon monoxide and/or carbon produced. In addition, such method may prevent reverse water-gas shift reaction (the following formula (c)) where a product hydrogen is oxidized into water, proactively preventing the loss of catalytic activity due to the reaction of catalyst and water (catalyst poisoning) and effectively supplying hydrogen necessary for methanation process. It can also be said that there are advantages of converting into gaseous CO the remaining carbon in the reaction furnace for cleaning up, and adapting to the case of producing CO or hydrogen as well as the case of simply conducting methanation over the case of simply conducting methane pyrolysis.

CO₂ + H₂ → CO + H₂O (c)

In the method of producing methane or propane, it is preferable to further satisfy the following condition (I) or (II):
(I) further comprising a step (5A) of using methane or propane produced in the step (4) as hydrocarbons for the step (1); or
(II) further comprising a step (5B) of using an unreacted material of hydrocarbons used in the step (2) for the step (1).

In either case, the supply amount of hydrocarbons may be reduced.

In the method of producing methane or propane, it is preferable that said step (2) may be a step of producing carbon and molecular hydrogen by the pyrolysis of methane 1.5 times or more the amount of CO₂ or propane 5/6 time or more the amount of CO₂ to be cyclically used in molar ratio. Such method makes it unnecessary to supply renewable energy consistently in the methanation process and makes it sufficient to use only methane or propane as a raw material in producing methane or propane and using it while achieving carbon neutrality. Furthermore, methane cost may be reduced by selling a part of carbon monoxide and/or carbon produced.

The method of producing methane or propane may comprise the step (4') of reacting CO₂ produced in the above step (1) with molecular hydrogen produced in the above step (1) or step (2) to produce methane or propane, instead of the step (4). Such method makes it sufficient to use an amount of methane to be pyrolyzed 2 times the amount of CO₂ or propane to be pyrolyzed 2.5 times the amount of CO₂, respectively, in molar ratio, which makes the step (3) of converting CO₂ into CO unnecessary, and thus simplifies the apparatus when compared to converting CO₂ into CO.

In the method of producing methane or propane, it is preferable that an amount of methane required for the pyrolysis of methane in said step (2) is 3 times or more the amount of CO₂ to be cyclically used in molar ratio and a remaining amount of methane produced in said step (4), i.e. an amount exceeding the amount of methane consumed by the oxidation reaction, is used for the step (2). Such method may reduce methane necessary for producing 1 Nm³ methane and using it while achieving carbon neutrality down to 2 times the amount of CO₂ (2 Nm³ at the minimum) in molar ratio. For the sake of ease, In terms of methane cost, 2 Nm³ of necessary methane amounts to 45.2 *2=90.4 yen/2Nm³, given the CIF price of 1 Nm³ methane amounts to 45.2 yen/Nm³·CH₄ (non-Patent Literature 9, non-Patent Literature 10; wherein the heat values of LNG and hydrogen are low heat values (LHV)) calculated on the premise that LNG be methane and the hydrogen cost equivalent to heat value of LNG be 13.3 yen/Nm³·H₂ (non-Patent Literature 8), which drastically reduces the cost compared to the cost for 4 Nm³ hydrogen necessary for the conventional method where hydrogen is supplied for methanation: 4*30 yen=120 yen/4 Nm³ (@2030). Furthermore, the offsite sourcing of hydrogen may generate the additional cost for ground transportation of hydrogen and receiving facilities. Therefore, the above method may be economically advantageous in terms of running cost. Such method may reduce the cost for 2 Nm³ of methane from 81.6 *2=163.2 yen/2Nm³ to 11 yen by further selling a part of produced carbon, given the CIF price of 1 Nm³ methane (81.6 yen/Nm³·CH₄; wherein the heat values of LNG and hydrogen are low heat values (LHV)) calculated on a premise that the hydrogen cost equivalent to heat value of LNG be 24 yen/Nm³·H₂ (non-Patent Literature 7). Such method may be economically advantageous even if compared to the cost for 4 Nm³ hydrogen necessary for the conventional method where hydrogen is supplied for methanation: 4*20 yen=80 yen/4 Nm³ (@2050).

In the method of producing methane or propane, it is preferable to conduct the reaction of said step (3) in the presence of carbon produced in the reaction of said step (2). This allows us to effectively consume and utilize produced carbon as a raw material of CO when it is not collected.

### [Advantageous Effects of Invention]

The method of producing CO or synthesis gas of the present invention may produce H₂ and CO separately from the reaction system without co-producing water. Therefore, H₂ and CO may be used separately, otherwise freely adjusting H₂/CO ratio depending on the target product in the case of producing synthesis gas.

Regarding the methane pyrolysis reaction, a consideration is given to the cost recovery for the effective use of the co-produced "solid carbon" in addition to hydrogen gas. The reaction apparatus of the present invention may offer great flexibility to introduce CO₂ into a reaction furnace to take place Boudouard reaction for the CO synthesis when the price of solid carbon is low.

According to the method of producing methane or propane of the present invention, such method makes it sufficient to use only hydrocarbons as a raw material when methane or propane is produced in the methanation/propanation process and uses it while achieving carbon neutrality. Furthermore, hydrocarbon cost may be reduced by selling a part of produced carbon.

### [Brief Description of the Drawing(s)]

[FIG. 1] (a) A cross-sectional view taken along the vertical direction and (b) A-A cross-sectional view of the reaction apparatus of Experimental Example 1.
[FIG. 2] A graph showing a time course of hydrogen concentration of Experimental Example 1.
[FIG. 3] A measurement result of SEM image and EDS of the catalyst to which a product is attached.
[FIG. 4] A graph showing the relationship between the temperature in a furnace and the equilibrium concentrations of CO and CO₂.
[FIG. 5] A graph showing a time course of CO and CO₂ concentrations after switching from N₂ gas to CO₂.
[FIG. 6] A schematic drawing showing an overview of one embodiment of CO₂ cyclic usage system installed onsite and the chemical species fed into/discharged from each module in a case where produced methane is (a) served as or (b) not served as a raw material of SDRM module.
[FIG. 7] A schematic drawing of the SDRM process.
[FIG. 8] A schematic drawing showing an overview of one embodiment of CO₂ cyclic usage system installed offsite.
[FIG. 9] A schematic drawing showing an overview of another embodiment of CO₂ cyclic usage system installed onsite and the chemical species fed into/discharged from each module in a case where produced methane is served as a raw material of SDRM module and hydrogen from renewable energy is also introduced.
[FIG. 10] A schematic drawing showing an overview of another embodiment of CO₂ cyclic usage system installed onsite and the mass balance of each module in a case where hydrogen is produced by methane decomposition module.
[FIG. 11] A schematic drawing showing an overview of another embodiment of CO₂ cyclic usage system installed onsite and the mass balance of each module in a case where propane is used for combustion and recycled for cyclic usage.
[FIG. 12] A schematic drawing showing an overview of another embodiment of CO₂ cyclic usage system installed onsite and the mass balance of each module in a case where propane is used for combustion and methane is recycled for cyclic usage.
[FIG. 13] A schematic drawing showing the mass balance of each module of CO₂ cyclic usage system installed onsite where a part of unreacted material is used for combustion given the methane decomposition rate be α.

### [Description of Embodiments]

Hereinafter, the definitions of "terms" used herein are given.

"Hydrocarbons" used herein is a linear or branched saturated hydrocarbons with a carbon number of 1 to 12 including methane, ethane, propane and octane, and a linear or branched unsaturated hydrocarbons with a carbon number of 1 to 12 including ethylene, propylene and acetylene.

"Substantially in the anhydrous condition" used herein means "in an environment where neither water nor steam is deliberately included or fed into a reaction system for the purpose of being involved with the reaction". Therefore, it is acceptable "substantially in the anhydrous condition" that water is incidentally or inevitably mixed by the reactions of raw materials and impurities or the reactions of plural impurities. It is desirable, however, to decrease moisture or humidity of raw materials by the contact with desiccant. "Proceeding/conducting reactions in a sequential order" or "reactions are conducted in a sequential order" used herein means proceeding/conducting plural reactions sequentially and/or alternatingly, not simultaneously, toward a desirable direction in case of a reversible reaction.

"Structural catalyst" used herein means a catalyst in which a structural body itself functions as a catalyst, or a catalyst with a structural body as a support. "A catalyst with a structural body as a support" generally refers to a catalyst obtained by immersing a substrate having a shape such as honeycomb into a slurry comprising a catalytic component, however, it may include the one obtained by forming an exposed, unsupported catalyst layer (plated layer, sprayed layer) by plating or spraying.

"Structural body" used herein means an object that has a certain shape without a mold to hold a shape. A shape of structural body may include, but be not limited to, for example, plate (not only a flat plate, but also includes the one obtained by subjecting the flat plate to any processing, such as folding, punching, notching, embossing etc.), stick, cylinder, porous body, honeycomb (monolith type), felt, mesh, fabric or expand metal etc.

"Carbon dioxide collection/storage (CCS) technique" used herein means a technique of collecting CO₂ exhausted from power plant or chemical plant before discharging into atmosphere, or collecting carbon dioxide from atmosphere for the storage or the effective utilization of CO₂ as a resource.

"Renewable energy" used herein means any energy capable of permanently utilizing as an energy source among non-fossil energy sources (Act on Rationalizing Energy Use, Act on the Promotion of Use of Non-Fossil Energy Sources and Effective Use of Fossil Energy Materials by Energy Suppliers, Article 2(3)). Such renewable energy may include, but not limited to, seven types: (a) solar photovoltaics, (b) wind power, (c) hydraulic power, (d) geothermal, (e) solar heat, (f) atmospheric heat and other heat present in nature, (g) biomass (organics derived from flora and fauna) (Enforcement order of the Act, Article 4).

"Chemical products" used herein means any product produced by chemical engineering with synthesis gas. Chemical products may include, for example, industrial chemicals such as methacrylic acid, methanol, acetic acid and aldehydes; synthetic fuels; chemical fertilizers; synthetic fibers; rubbers; synthetic resins such as polycarbonates and urethanes; pharmaceuticals; dyes; soaps; and cosmetic products.

One embodiment of the present invention is a method of producing carbon monoxide or carbon monoxide and molecular hydrogen, comprising the steps of proceeding the reactions represented by the following formulae (1) and (2) in a sequential order within the identical container substantially in the anhydrous condition:

CH₄ → C + 2H₂ (1)

C + CO₂ → 2CO (2)

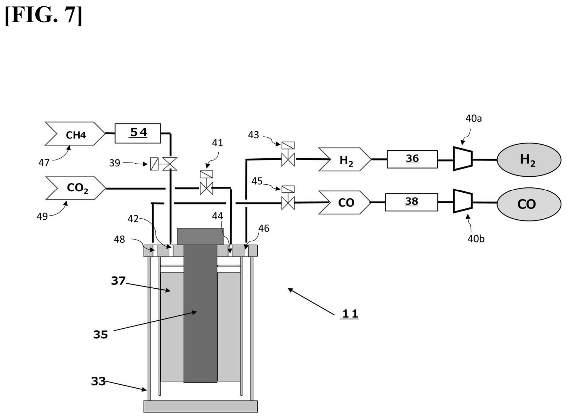

Unless otherwise explained, the specific conditions of the reactions represented by the above formulae (1) and (2) as well as the catalyst design are not described hereinafter since these are the same as the matters described in the following about the steps (2) and (3) of the production method of methane or propane according to one embodiment of the present invention. Furthermore, the specific examples of the apparatus design are not described since these are similar to the SDRM reaction module 11 as described in the following with reference to FIG. 7.

If the present invention is taken as a method of producing carbon monoxide, there is no limitation to the handling of carbon or hydrogen produced in the above formula (1). Specifically, it may be used or not be used for the subsequent reaction involved with carbon monoxide, it may be used solely, or be disposed of.

Even if the present invention is taken as a method of producing carbon monoxide, carbon monoxide may include hydrogen or methane or the other impurities. In proceeding the latter-stage reaction (formula (2)) of the production method, it depends on the required purity of produced CO, i.e. the use of CO, whether to necessitate the removal of hydrogen or methane remained in the former-stage reaction (formula (1)). It is preferable, however, to proceed the reactions represented by the aforesaid formulae (1) and (2) in a sequential order substantially in the oxygen-free environment (in an environment free from O₂).

If the present invention is taken as a method of producing carbon monoxide and hydrogen, it is not limited to the production method of synthesis gas consisting of carbon monoxide and hydrogen. The present invention may encompass the case where the use of carbon monoxide and the use of hydrogen are totally different from each other. The use of carbon monoxide other than synthesis gas may be the reducing agent of metal oxide etc. The use of hydrogen other than synthesis gas may be a fuel to proceed the reaction of the aforesaid formula (1) or (2), the admixture with city gas, turbine fuel, a raw material of fuel cell etc.

Methane (CH₄) used in the aforesaid formula (1) may be preferably from natural gas. Natural gas may contain methane (CH₄) in high concentration and have good supply. City gas derived from natural gas may also easily become a methane source by passing through a desulfation device. Natural gas may include city gas, non-associated gas produced from gas field, associated gas produced from oil field together with crude oil, shale gas and methane hydrate etc. Methane may be from biogas, i.e. methane obtained by separating CO₂ from biogas in view of the global trend of carbon negative, however, it has poor supply. Methane from coal gas may be used, however, coal gas is a mixed gas in nature, and thus there might be a disadvantage of failing to supply methane and CO₂ separately.

It may be preferable that carbon dioxide (CO₂) is CO₂ stored by the carbon dioxide collection/storage (CCS) technique. CO₂ stored by the carbon dioxide collection/storage (CCS) technique may be the one that has already been stored and injected deep underground, or the one that has temporarily stored on the ground in preparation for storage and injection, or the one that has just been separated from the other gases. CO₂ may be from biogas, i.e. CO₂ obtained by separating methane from biogas, however, it has poor supply.

Another embodiment of the present invention is a method of producing a synthesis gas or a chemical product using the same, comprising the step of mixing carbon monoxide and molecular hydrogen obtained by said method in a certain ratio by the molar flow rate adjustment.

The molar flow rate adjustment refers to the flow rate adjustment between carbon monoxide and hydrogen on a molar ratio basis with the stoichiometric ratio of hydrogen and carbon monoxide of the subsequent reactions in mind by use of synthesis gas, an admixture gas of hydrogen and carbon monoxide. The certain ratio used herein may not always be accurately equal to the stoichiometric ratio. On the basis of the findings established or to be established with regard to the subsequent reactions by use of synthesis gas, operation conditions and the ratio of carbon and carbon monoxide as side products of hydrogen, it is permitted to lower or increase the certain ratio compared to the stoichiometric ratio. The certain ratio may be set to e.g. ±50%, ±20% and ±5% etc. higher/lower compared to the stoichiometric ratio of the subsequent reaction.

The method of producing a synthesis gas or a chemical product may further comprise the step of adding molecular hydrogen from renewable energy to adjust a H₂/CO ratio. Hydrogen derived from renewable energy may include, but be not limited to, for example, hydrogen generated by the electrolysis of water for which the excess power of solar power/wind power generation is used.

The production method of methane or propane according to one embodiment of the present invention is a method of producing methane or propane by cyclic usage of CO₂ produced following oxidation reaction of hydrocarbons.

"Cyclic usage of CO₂" used herein means that an amount of methane equimolar to/an amount of propane 1/3 times of the amount of CO₂ generated may be ultimately produced or secured. Therefore, in order to achieve "the cyclic usage" used herein, it is not required to combust or reuse produced methane or propane in a plant site, reuse it as a raw material for SDRM reaction module or methane oxidation module, or use it as heat source of SDRM reaction module or methane oxidation module by combustion, but it is sufficient to use it for the other use by supplying to city gas pipeline or tank.

The production method of methane or propane according to one embodiment of the present invention comprises the step (1) of producing CO₂ following oxidation reaction of hydrocarbons.

"Following oxidation reaction of hydrocarbons" used herein means "by way of any one-step or plural steps of reaction(s) involving any reaction where hydrocarbon(s) is/are considered as a reducing agent and one or two or more other chemical substance(s) is/are considered as an oxidizing agent. Such reaction may not be limited to the combustion reactions of hydrocarbons represented by the following formula (d), formula (e) and formula (f), but also include the steam reforming reactions of hydrocarbons represented by the following formula (g) and formula (h).

CₙH₂ₙ₊₂ + ((3n+1)/2)O₂ → nCO₂ + (n+1)H₂O • • • • (d)

CₙH₂ₙ + (3n/2)O₂ → nCO₂ + nH₂O • • • • (e)

CₙH₂ₙ₋₂ + ((3n-1)/2)O₂ → nCO₂ + (n-1)H₂O • • • • (f)

CH₄ + H₂O → CO + 3H₂ .... (g)

CO + H₂O → CO₂ + H₂ • • • • (h)

The oxidation reaction of hydrocarbons may be exoergic (combustion) reaction or endoergic reaction, and may/may not require the use of catalyst, depending on the reactions involved.

In the combustion reactions of the above formula (d), formula (e) and formula (f), catalyst may not be used. However, Pt/SnO₂-based catalyst, Pt-Ir/SnO₂-based catalyst or Co/Pd bearing support catalyst may be an example of the catalyst in the case where it may be used for the complete combustion of the remaining methane in exhaust gas.

The reaction temperature of the aforesaid formulae (d), (e) and (f) may be usually set to 340°C to 500°C in the presence of catalyst.

Catalyst for the use in steam reforming reaction of the above formula (g) may include the ones in which the catalytically active component such as nickel, cobalt, molybdenum, rhodium, ruthenium, aluminum, zirconium, magnesium and oxides thereof is supported on a porous support. Furthermore, specific examples may include Ru/Al₂O₃ catalyst in which ruthenium is supported on aluminum support and Ni/Al₂O₃ catalyst in which nickel is supported on aluminum support.

The reaction temperature of the aforesaid formulae (g) and (h) may be usually set to 500 to 900°C in the presence of catalyst.

The supply source of hydrocarbons to be oxidized is preferably from oil field, crude oil, natural gas, city gas or biogas. The supply source of methane is preferably from natural gas, city gas or biogas. City gas may contain methane (CH₄) in high concentration and be easily available from the existing pipeline network. City gas derived from natural gas may also easily become a methane source by passing through a desulfation device. In view of the global trend toward carbon negative, the example of the supply source may include carbon-neutral methane from natural gas or carbon-neutral methane from biogas, i.e. methane obtained by separating CO₂ from biogas or CO₂-containing methane.

The oxidizing agent for combustion may include air, pure oxygen and oxygen-enriched air in which oxygen is added to air. Pure oxygen may be from pure hydrogen and pure oxygen that are produced by water electrolysis conducted by renewable energy such as solar power (installed on a plant site) as well as purchasing as industrial gas. Alternatively, pure oxygen may be from pure hydrogen and pure oxygen that are produced by the water electrolysis apparatus (installed on a plant site) driven by renewable energy purchased from grid power. Additionally, pure hydrogen produced by the water electrolysis apparatus may be utilized as a fuel of heating apparatus 35 for pyrolysis mentioned below.

The production method of methane or propane according to one embodiment of the present invention may comprise a step (2) of subjecting methane or propane prepared separately from hydrocarbons to be oxidized to pyrolysis through the methane pyrolysis reaction represented by the following formula (i) or the propane pyrolysis reaction represented by the following formula (j) substantially in the anhydrous condition to produce carbon and molecular hydrogen.

CH₄ → C + 2H₂ (i)

C₃H₈ → 3C+4H₂ (j)

The reaction temperature of the aforesaid formula (i) or (j) may be usually set to a high temperature of 1000°C to 1200°C without catalyst and usually set to 600°C to 800°C or higher in the presence of catalyst. The reaction temperature used herein may be a value obtained by the measurement with thermocouples.

The reaction represented by the above formula (i) or formula (j) may proceed with or without catalyst, however, when proceeding at the above reaction temperature, it is preferable to use catalyst.

The reaction of the aforesaid formula (i) or formula (j) may be conducted usually in the presence of catalyst having a catalytic activity on methane pyrolysis reaction. A catalyst "having a catalytic activity on methane pyrolysis reaction" may preferably comprise a support layer having a thickness of 0.5 mm to about 10 mm and an exposed nickel-containing layer having a thickness of 1 µm to 200 µm, said support layer consisting of iron, copper, nickel, steel, cast iron, iron nickel alloy or copper alloy, and more preferably comprise an interlayer comprising copper and having a thickness of 1 to 1000 µm between the support layer and the nickel-containing layer. The support layer may be a structural body itself before coating a nickel-containing layer, or a layer coated on a structural body. "Exposed" used herein may be enough if methane molecule may contact and not be limited to the one that can be seen as exposed by sight. Nickel-containing layer may be an unsupported nickel-containing layer. "Unsupported" means that a catalytic component of nickel-containing component is present in a structurized manner, not present as a particle distributed on a porous support such as active carbon and porous oxides. "Structurized" may include a state where particles are welded in a partial region, a state where particles are welded in a whole region, or a state where particles are melted as a whole and then cooled and solidified.

Furthermore, the catalyst "having a catalytic activity on methane pyrolysis reaction" may not be limited to a structural body catalyst, but also include a powder catalyst that cannot retain a certain shape without a mold to hold a shape.

Methane (CH₄), separately prepared from hydrocarbons to be oxidized used in the above formula (i) or (j), is preferably derived from city gas. City gas may contain methane (CH₄) in high concentration and be easily available from the existing pipeline network. City gas derived from natural gas may also easily become a methane source by passing through a desulfation device. Methane may be from biogas, i.e. methane obtained by separating CO₂ from biogas in view of the global trend of carbon negative, however, it has poor supply. Methane from coal gas may be used, however, coal gas is a mixed gas in nature, and thus there might be a disadvantage of failing to supply methane and CO₂ separately.

The amount of methane for pyrolysis is usually 1.5 times or more the amount of CO₂ to be cyclically used in molar ratio. Strictly speaking, however, it is permitted to lower or raise the lowest value of 1.5 times on the basis of the findings established or to be established with regard to the subsequent reactions by use of synthesis gas, operation conditions and the ratio of carbon and carbon monoxide as side products of hydrogen. The lowest value of molar ratio may be preferably 1.8 times, 2 times, 2.5 times and 3 times the amount of CO₂ to be cyclically used.

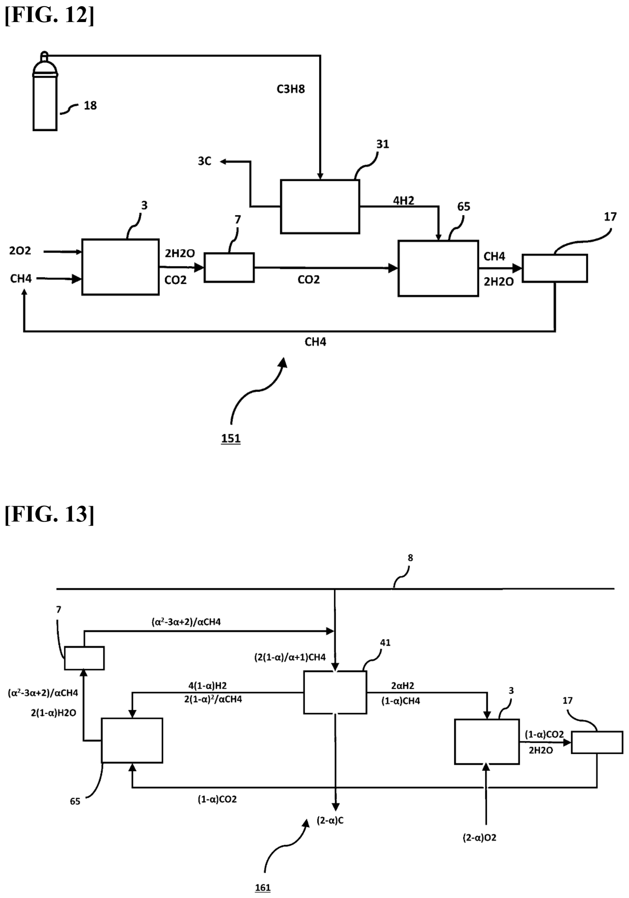

The amount of propane for pyrolysis is usually 5/6 time or more the amount of CO₂ to be cyclically used in molar ratio. Strictly speaking, however, it is permitted to lower or raise the lowest value of 5/6 time on the basis of the findings established or to be established with regard to the subsequent reactions by use of synthesis gas, whether to produce methane or propane in the subsequent reaction (the lowest molar ratio when methane is produced is stoichiometrically 1 time as shown in FIG.12), operation conditions and the ratio of carbon and carbon monoxide as side products of hydrogen. The lowest value of molar ratio may be preferably 1 time, 2 times, 2.5 times and 3 times the amount of CO₂ to be cyclically used.

The molar ratio may be set to e.g. ±50%, ±20% and ±5% etc. higher/lower compared to the lowest value.

The production method of methane or propane according to one embodiment of the present invention may comprise a step (3) of conducting Boudouard reaction represented by the following formula (k) in a sequential order substantially in the anhydrous condition, producing carbon monoxide by reacting CO₂ produced following oxidation reaction of hydrocarbons with carbon produced in the above step (2):

C + CO₂ → 2CO (k)

The reaction temperature of the above formula (k) may usually be set to 400°C or higher in the presence of catalyst, preferably 650°C or higher to proceed with the reaction in favor of CO product side, more preferably higher than 650°C, further preferably 800°C or higher.

The reaction represented by the above formula (k) may proceed with or without catalyst, however, when proceeding at the above reaction temperature, it is preferable to use catalyst.

The aforementioned reaction of formula (k) may be usually conducted in the presence of carbon produced in said reaction of formula (i) or formula (j).

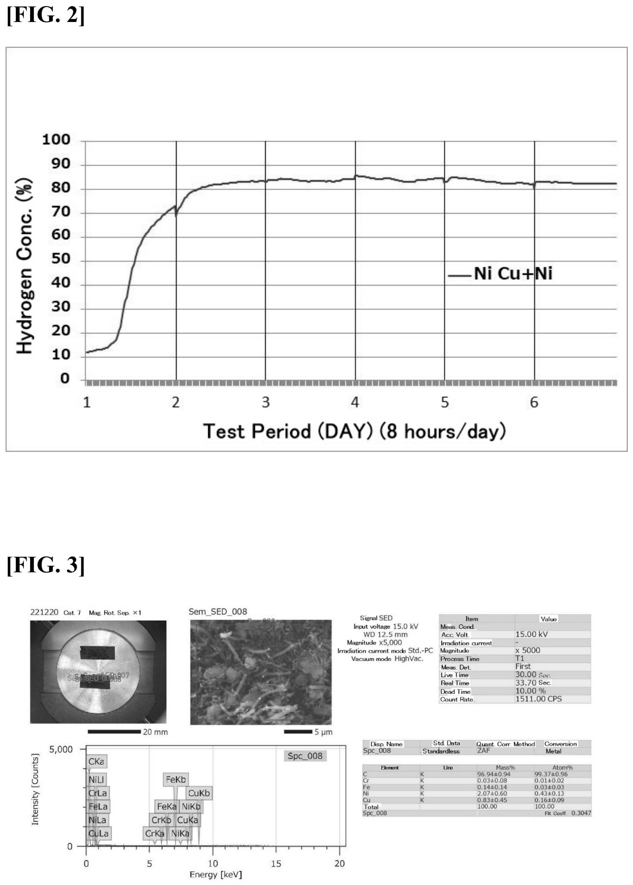

It is enough to leave some amount of carbon produced herein. Partial amount of carbon may be removed. A major amount of carbon produced herein may be observed as nanofibers (tip-growth form with a nickel nanoparticle at the tip as shown as a white point in SEM image of FIG. 3). Produced carbon containing these catalyst fineparticles not only serves as a raw material, but also possibly has a catalytic activity. No matter in what form or role it is present, it is enough to leave the produced carbon in conducting the production method of the present invention. Specifically, it should be noted that what has a catalytic activity does not constrain the technical scope of the production method of the present invention.

Of the carbon produced in the step (2), the excess portion that was not used in the step (3) may be converted into a valuable product for sale, thereby reducing the methane procurement cost. Although no one can provide a general discussion due to the difference in the shape of carbon produced (sphere-shaped, fibre-shaped), depending on the kinds of the structural catalyst and the reaction conditions, the electroconductivity of produced carbon is comparable to that of Ketjenblack in the case where spheroidal graphites with different particle sizes are produced. Thus it can be sold as it is, or as carbon black after removing metal fineparticles (The price of carbon black is 142 yen/kg (76 yen/Nm³·CH₄@2020), reference URL: https://www.meti.go.jp/statistics/tyo/seidou/result/gaiyo/resourceData/02_kagaku/nenpo /h2dbb2020k.pdf#page=13). Produced carbon may also be mixed with thermoplastic resin PPS and then sold as a high-thermal conductivity resin, or may be sold as carbon additive in a casting process, but the use is not particularly limited.

Of the carbon monoxide produced in the step (3), if there is any excess portion not used in the subsequent step (4), it may be used, as necessary, for casting, acrylic acid production, gaseous fuels, or the reductant for metal oxides, calcium oxide, calcium phosphate or silicon dioxide.

The production method of methane or propane according to another embodiment of the present invention may use CO₂ produced in the step (1) for the step (4) without implementing the step (3). Whether to implement the step (3) depends on the condition of the methane pyrolysis reaction.

The method of producing methane or propane according to one embodiment of the present invention may produce methane by proceeding with the Fischer-Tropshe reaction as represented by the following formula (1) in which hydrogen produced in the above step (2) with CO produced in the above step (3), or comprise the step (4) of reacting CO₂ produced in the above step (1) with molecular hydrogen produced in the above step (1) or step (2) without implementing the step (3), as represented by the following formula (m):

3H₂ + CO → CH₄ + H₂O (l)

4H₂ + CO₂ → CH₄ + 2H₂O (m)

The reaction temperature of the above formulae (1) and (m) may usually be set to 100°C to 300°C in the presence of catalyst. A condition of low temperature and high pressure is advantageous to avoid the runaway reaction and proceed with the reaction in favor of methane product side, whereas the temperature elevation is required to develop catalytic activity and increase the CO conversion rate. Thus the upper limit is preferably 260°C, more preferably 250°C. The preferable lower limit is 130°C, more preferably 170°C. To achieve the above reaction temperature, one may use excess waste heat after heating to proceed the reactions of the above formulae (1) and (m).

The reactions represented by the above formulae (1) and (m) may proceed with or without catalyst, however, when proceeding at the above reaction temperature, it is preferable to use catalyst. Usable catalysts may include, for example, those obtained by supporting at least one element selected from the group consisting of iron, ruthenium, rhodium, nickel, and cobalt as an active component on a carrier material selected from the group consisting of Al₂O₃, ZrO₂, TiO₂, SiC, SiO₂, ZnO, oxides of Group IIIA metals, oxides of transition metals of Groups IIIB, IVB, VB, and VIB, oxides of rare earth metals, aluminosilicates, zeolites, MOFs (metal-organic frameworks), and mixtures thereof, as well as catalysts having catalytic activity for the above-mentioned methane pyrolysis reaction. Examples of the commercial products may include publicly-known catalyst used for the removal of carbon monoxide by methanation such as N110 series (manufactured by JGC Catalysts and Chemicals Ltd.), core-shell type catalyst (manufactured by IHI) in which Ni particle is dispersed into a porous matrix described in Patent No. 6203375, core-shell type catalyst (MITSUI MINING & SMELTING CO., LTD.) consisting of Ni-containing core and Zirconium-containing shell as described in the International Publication No. 2022/065468.

In usual, hydrogen produced in the above step (1) or step (2) may be used as a supply source of hydrogen. In shortage, hydrogen from water electrolysis using renewable energy may be utilized.

Specific system configuration will be discussed hereinafter when methane is used as hydrocarbons.

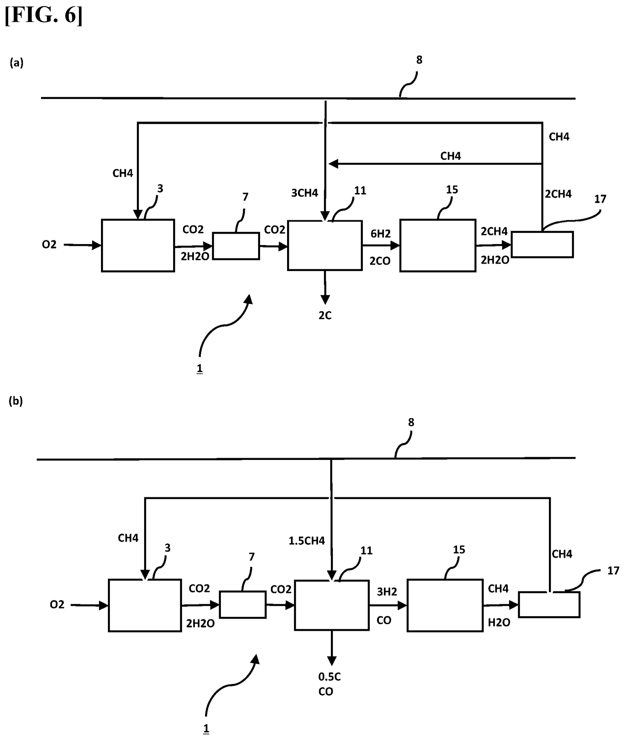

CO₂ cyclic usage system 1 as shown in FIG. 6 is a system mainly intended for the installation on the sites of city gas consumers and biogas producers, comprising: methane oxidation module 3, dehydration device 7 to remove H₂O from mixed gas of CO₂ and H₂O discharged from methane oxidation module 3, SDRM reaction module 11 to receive methane supplied from pipeline 8 and CO₂ and city gas discharged from dehydration device 7, methanation module 15 to supply H₂ and CO as raw materials discharged from SDRM reaction module 11 and discharge CH₄ and H₂O, and dehydration device 17 to remove H₂O from mixed gas of CH₄ and H₂O.

Methane oxidation module 3 is a device or components thereof to oxidize methane and discharge CO₂. Examples of methane oxidation module 3 may include publicly-known gas turbine generator, combined cycle generator, diesel generator, biogas generator, gas turbine for pump drive, boiler, chiller, absorption hot and chilled water generator, solid-oxide fuel cell (Ene-farm) and steam reformer etc.

Dehydration device 7 may remove moisture when water is produced in methane oxidation module 3 via the oxidation reaction of methane (e.g. when oxygen is used as an oxidizing agent) or water is used as an oxidizing agent of methane, thereby avoiding catalyst poisoning etc. due to the admixing of water into the subsequent SDRM reaction module 11.

Dehydration device 17 may remove moisture co-produced with methane in methanation module 15, thereby avoiding a decline in thermal efficiency due to the admixing of water into the subsequent methane oxidation module 3 or catalyst poisoning etc. due to the admixing of water into the subsequent SDRM reaction module 11.

Dehydration devices 7, 17 may adopt a method of cold condensation, a method of separation using the membrane such as polyimide membrane, ceramic membrane and zeolite membrane, a method of using absorbent such as silica gel, molecular sieve, zeolite and active carbon.

Additionally, when it is permitted to decline the oxidation efficiency or the conversion rate, or when the improved catalyst is used, the dehydration devices 7 and 17 may be omitted.

Methanation module 15 refers to a device or components thereof to which H₂ and CO discharged from SDRM reaction module 11 are supplied and from which CH₄ and H₂O are discharged. Methane synthesis unit installed downstream of SOEC methanation may be served as methanation module 15.

SDRM reaction module 11 as shown in FIG. 7 is provided with: reaction furnace 33 having methane gas inlet 42, CO₂ supply inlet 44, hydrogen gas outlet 46 and CO gas outlet 48; heating apparatus 35 inserted from the top part of reaction furnace 33 into the furnace and fixed; structural catalyst 37 having a catalytic activity of methane pyrolysis arranged surrounding heating apparatus 35 in reaction furnace 33; first valve 39 controlling the supply of CH₄ from CH₄ supply source 47 to methane gas inlet 42; second valve 41 controlling the supply of CO₂ from CO₂ supply source 49 to CO₂ gas inlet 44; third valve 43 controlling the discharge of hydrogen from hydrogen gas outlet 46; and fourth valve 45 controlling the discharge of CO from CO gas outlet 48.

Heating apparatus 35 may adopt the internal heating method to heat reaction furnace 33 from the inside by inserting into reaction furnace 33. Specifically, recuperative burner is adopted with hydrogen as a fuel. Instead, biogas may be used as a fuel. (External heating method) heater for heating reaction furnace 33 from the outside may also be adopted.

Structural catalyst 37 having a catalytic activity of methane pyrolysis reaction may be obtained by electroplating copper layer as an interlayer containing copper and nickel layer as a nickel-containing layer in this order on nickel-plate catalyst as a support layer (structural body).

First to fourth valves 9, 41, 43, 45 may be gate valves capable of switching between full-open to full-close. Instead, any other valve such as ball valve can be used. Butterfly valve or glove valve may be used particularly when the flow adjustment is required. The switching operation of opening/closing valve is done manually, or done automatically by use of electromagnetic valve etc.

Desulfation device 54 may be installed between CH₄ supply source 47 and first valve 39 to remove a trace amount of sulfur-based compounds contained in city gas. Desulfation device 54 may be a publicly-known solid catalyst or an absorbent.

Pressure Swing Absorption (PSA) method may be adopted for H₂ purification apparatus 36. Instead, one can adopt any other purification method permeating through zeolite membrane, DDR membrane and palladium alloy membrane etc.

CO purification apparatus 38 may be a replaceable solid absorbent made of amine-supported porous support capable of absorbing CO₂. Instead, one can use liquid absorbent, membrane separation method and PSA method.

Purification apparatuses 36, 38 may not be installed when required purity is low.

CO detector not shown in the figure may be installed on the path connecting gas outlet of reaction furnace to fourth valve 45 or the path connecting fourth valve 45 to CO purification apparatus 38. CO detector is an instrument capable of specifically detecting the carbon monoxide concentration or an indicator reflecting the change of the concentration (e.g. potential change or light absorption change etc.). In the present embodiment, CO detector may be NDIR gas sensor, but the measurement method is not particularly limited. Detector may also detect the concentration of gas other than carbon monoxide.

Compressors 40a, 40b may be installed respectively downstream of purification apparatuses 36, 38.

The reaction module is practically operated with second valve 41 and fourth valve 45 being closed when first valve 39 and third valve 43 are open, and with first valve 39 and third valve 43 being closed when second valve 41 and fourth valve 45 are open. Regarding the procedure to open/close each valve, methane pyrolysis reaction of formula (i) or propane pyrolysis reaction of formula (j) is conducted with second valve 41 being closed → fourth valve 45 being closed → first valve 39 being opened → third valve 43 being opened. CO₂ reducing reaction of formula (k) is conducted with first valve 39 being closed → third valve 43 being closed → second valve 41 being opened → fourth valve 45 being opened.

CO₂ cyclic usage system 1 having the aforementioned configuration may eliminate the need for solid oxide electrolysis cell apparatus or renewable power generation apparatus that was necessary for reducing CO₂ and/or water in methanation process, apparatus for reducing produced metal oxides that was necessary for reducing CO₂ and/or water with metal in methanation process, water electrolysis hydrogen-producing apparatus, hydrogen storage facilities and hydrogen pipes when hydrogen is produced from water in methanation process, instead of introducing SDRM reaction module 11. It may also eliminate the need for transportation vehicles, compressor, storage facilities and drivers on the supply side, storage facilities on the receiving side, all of which would be necessary if hydrogen were produced offsite and received onsite.

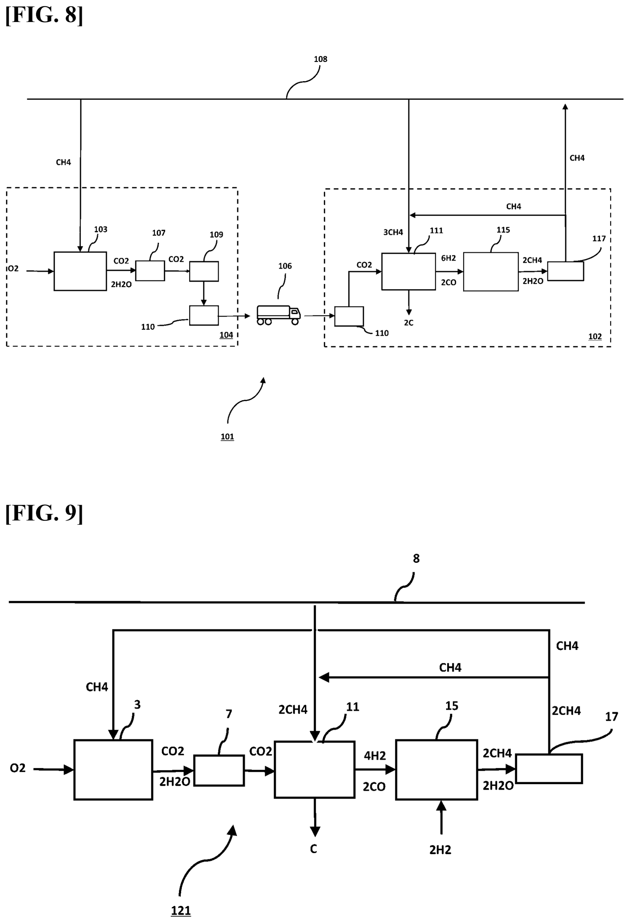

CO₂ cyclic usage system 101 as shown in FIG. 8 is a system mainly intended for the distributed installation off the site of city gas suppliers 102 and the site of city gas consumers 104, comprising: methane oxidation module 103 installed on the site of city gas consumers 104, dehydration device 107 to remove H₂O from mixed gas of CO₂ and H₂O discharged from methane oxidation module 103, transportation means 106 to transport CO₂ discharged from dehydration device 107 to the site of city gas suppliers 102, SDRM reaction module 111 installed on site of city gas suppliers 102 to receive CO₂ supplied from transportation means 106 and methane supplied from pipeline 108, methanation module 115 to supply H₂ and CO as raw materials discharged from SDRM reaction module 111 and discharge CH₄ and H₂O, and dehydration device 117 to remove H₂O from mixed gas of CH₄ and H₂O.

CO₂ cyclic usage system 101 having the aforementioned configuration may require CO₂ compressor/liquefier 109, CO₂ shipping facility (not shown), CO₂ receiving facility (not shown), CO₂ storage facilities 110, CO₂ tanker truck 106, driver (not shown), however, such system may have a similar advantage to CO₂ cyclic usage system installed onsite 1 over the methanation process that necessitates conventional hydrogen supply.

A difference of CO₂ cyclic usage system 121 as shown in FIG. 9 from CO₂ cyclic usage system 1 as shown in FIG. 6 is that hydrogen from renewable energy is supplied from outside resource to methanation module 15 in addition to H₂ discharged from SDRM reaction module 11.

CO₂ cyclic usage system 121 having the aforementioned configuration may have several advantages over CO₂ cyclic usage system 1 as shown in FIG. 6 of: reducing hydrogen from renewable energy to be supplied from outside resource from 4 unit volume to 2 unit volume, reducing cost from 30 yen/Nm³ (@2030) * 4 Nm³ = 120 yen to 60 yen, or 20 yen/Nm³ (@2050) * 4 Nm³ = 80 yen to 40 yen, reducing natural gas to be supplied from outside resource (city gas, methane) from 2 unit volume to 1 unit volume, reducing the amount of produced carbon by half and facilitating market cultivation and monetization of produced carbon.

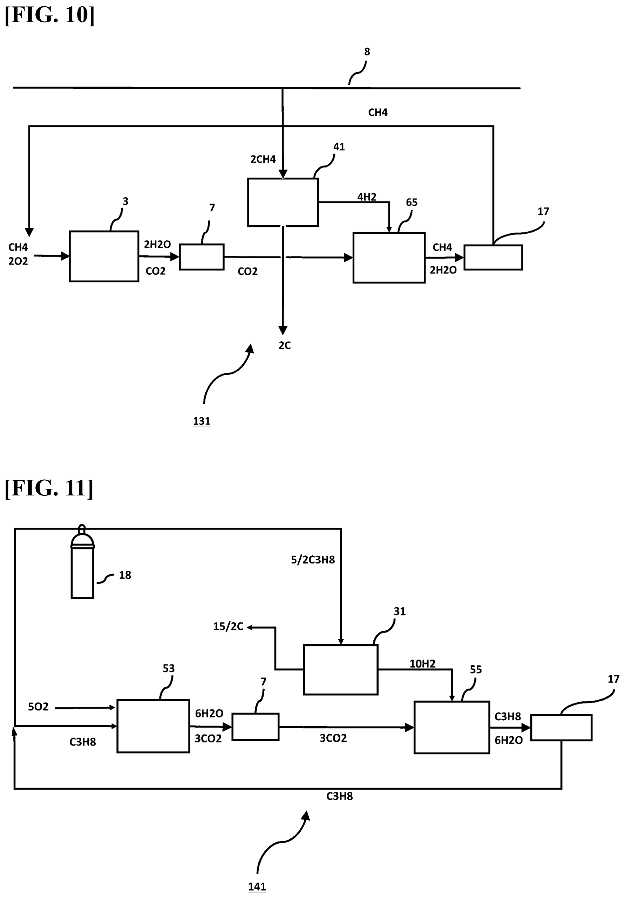

A difference of CO₂ cyclic usage system 131 as shown in FIG. 10 from CO₂ cyclic usage system 1 as shown in FIG. 6 is that methane pyrolysis module 41 is installed in place of SDRM reaction module 11 to supply produced hydrogen to methanation module 65, and supply CO₂ discharged from dehydration device 7 directly to methanation module 65. Structural catalyst 37 having a catalytic activity of methane pyrolysis reaction used in SDRM reaction module 11 may be used for methane pyrolysis module 41. Methanation module 65 may include core-shell type catalyst (MITSUI MINING & SMELTING CO., LTD.) consisting of Ni-containing core and zirconium-containing shell as described in the International Publication No. 2022/065468.

CO₂ cyclic usage system 131 having the aforementioned configuration may not require the conversion of CO₂ into CO, but only require the amount of methane for pyrolysis 2 times the amount of CO₂ to be cyclically used in molar ratio as compared to CO₂ cyclic usage system 1 as shown in FIG. 6. Therefore it is no longer necessary to install second valve 41 and CO₂ inlet 44 on the system for introducing CO₂, and CO gas outlet 48, fourth valve 45, purification apparatus 38 and compressor 40b on the system for discharging CO. Thus there is an advantage that the apparatus is more simplified compared to the apparatus where the conversion takes place.

A difference of CO₂ cyclic usage system 141 as shown in FIG. 11 from CO₂ cyclic usage system 131 as shown in FIG. 10 is that the methane supply from pipeline 8 to methane pyrolysis module 41 is abolished, and instead the propane supply from propane tank 18 to propane pyrolysis module 31, propanation module 55 is used in place of methanation module 65, propane from propane tank 18 and propane from propanation module 55 are utilized for propane oxidation module 53.

Propane oxidation module 53, propane pyrolysis module 31 and propanation module 55 may fundamentally have a configuration similar to methane oxidation module 3, methane pyrolysis module 41 and methanation module 65, respectively.

CO₂ cyclic usage system 141 having the aforementioned configuration may become suitable for producing propane through cyclic usage of CO₂ produced by the combustion of propane in a suburban plant without city gas pipeline as compared to CO₂ cyclic usage system 131 as shown in FIG. 10.

A difference of CO₂ cyclic usage system 151 as shown in FIG. 12 from CO₂ cyclic usage system 141 as shown in FIG. 11 is that methanation module 65 is used in place of propanation module 55 to circulate methane and use methane for combustion at methane oxidation module 3.

CO₂ cyclic usage system 151 having the aforementioned configuration may require a larger amount of propane in relative to CO₂ for cyclic usage when compared to CO₂ cyclic usage system 141 as shown in FIG. 11 (the former is 5/6 time or more the amount of CO₂, the latter is 1 time or more the amount of CO₂), however, use methane cyclically. Therefore, it discharges less CO₂ generated by combustion compared to propane, and thus advantageous from the viewpoint of CO₂ emission.

A difference of CO₂ cyclic usage system 161 as shown in FIG. 13 from CO₂ cyclic usage system 131 as shown in FIG. 10 is that methane pyrolysis module 41 is installed between pipeline 8 and methane oxidation module 3, not downstream of methane oxidation module 3, and the supply to methanation module 65 is remained, and recycled methane is returned to methane pyrolysis module 41.

CO₂ cyclic usage system 161 having the aforementioned configuration may exclusively burn hydrogen if methane decomposition rate is 1, and burn a mixture of hydrogen and the other gas if methane decomposition rate is less than 1, and thus require an excess amount of a mixture of methane and hydrogen to ensure the same calory as that of the conventional exclusive burning of methane, as compared to CO₂ cyclic usage system 131 as shown in FIG.10 (heat value of methane (LHV)=3 5900 kJ/Nm³, heat value of hydrogen (LHV)=10780 kJ/Nm³), and eliminate the need for the separation and purification of unreacted methane, and thus simplify the apparatus.

### (Experimental Example 1 - Manufacture of SDRM reaction module and methane pyrolysis)

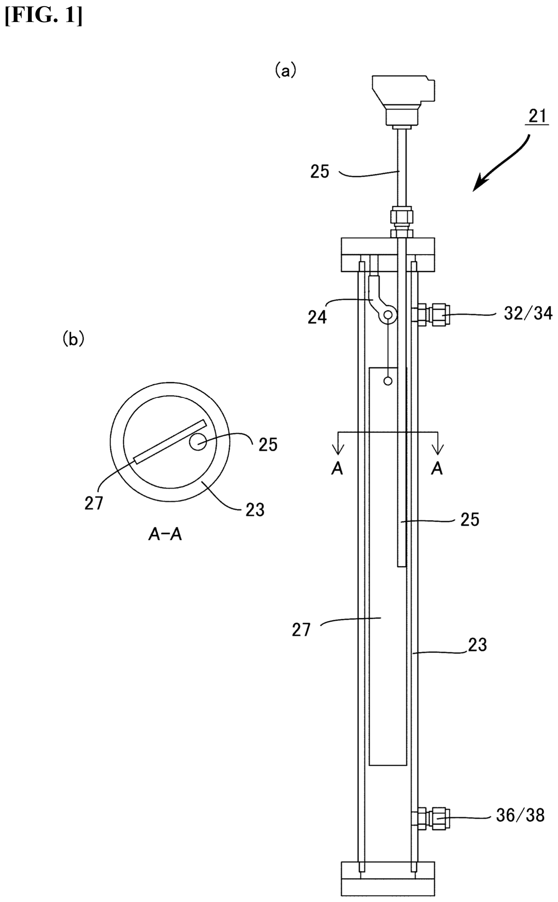

SDRM reaction module 21 of FIG. 1 comprises metal plate catalyst 27 (size: width 30 mm * length 300 mm * thickness 0.6 mm) hanging from catalyst hanger 24 in reaction furnace 23 (volume Φ30*300 mm, 0.2L), wherein the catalyst is obtained by subjecting both surfaces of Ni sheet to electroplating with Cu approximately in a thickness of 1 µm, and subjecting Cu-plated Ni sheet to electroplating with Ni approximately in a thickness of 10 µm. Flowing methane at room temperature at a flow rate of 10 ml/min from methane inlet 32 installed on the upper peripheral wall of furnace 23 from the top to the bottom, heated up to 800°C with heater (not shown) installed around the outer circumference of the furnace 23 and kept for 8 hours per day to observe the time course of hydrogen concentration discharged from hydrogen gas outlet 36. For safety, the furnace core was cooled after the continuous operation for 8 hours each day, and heated again up to 800°C from room temperature next day. A time course of hydrogen concentration is shown in FIG. 2 and the SEM image of the catalyst to which product is attached and the measurement result of EDS are shown in FIG. 3.

Furnace temperature was measured by thermocouple 25 penetrated through the top lid of the furnace and inserted into the center portion of the furnace. Hydrogen concentration was measured by thermal conductivity detector (zero gas: city gas 13A; span gas: hydrogen 100%; gas flow rate: 0.2 L/min, manufactured by CHINO CORPORATION) with Wheatstone bridge circuit attached to product gas discharging pipe open to the atmosphere inserted at the bottom part of peripheral wall of the furnace. SEM image and EDS were obtained by scanning electron microscope (Product #: JCM-7000, manufactured by JEOL) via secondary electron detection (input voltage: 15.0 kV; WD; 12.5 mm; magnitude: 5000 times; irradiation current mode: Std.-PC Vacuum mode: HighVac.) .

As can be seen from FIG. 2, hydrogen concentration (conversion rate) due to methane pyrolysis reaction was continued to elevate from Day 1 to Day 2. At the end of Day 2, hydrogen concentration was reached up to the theoretical equilibrium concentration of 80%, and stably stood at around 80% after Day 6.

From FIG. 3, the product was in fibril shape, and mainly composed of C and Ni. The study of elemental mapping implemented separately showed that white dots found at the tip or middle of fiber was Ni fine particles.

### (Experimental Example 2 - Demonstration of Boudouard reaction in the presence of produced carbon and the investigation of the reaction temperature)

Following Experimental Example 1, the supply of methane was stopped and the weight of produced carbon in reaction furnace was measured. Following the measurement of the weight of the produced carbon, metal catalyst plate to which produced carbon was attached and the produced carbon were returned again to the reaction furnace. To remove air taken into the reaction furnace, nitrogen gas was filled at a flow rate of 0.4 L/min for 30 minutes at room temperature in reaction furnace to purge methane and hydrogen remained in the furnace.

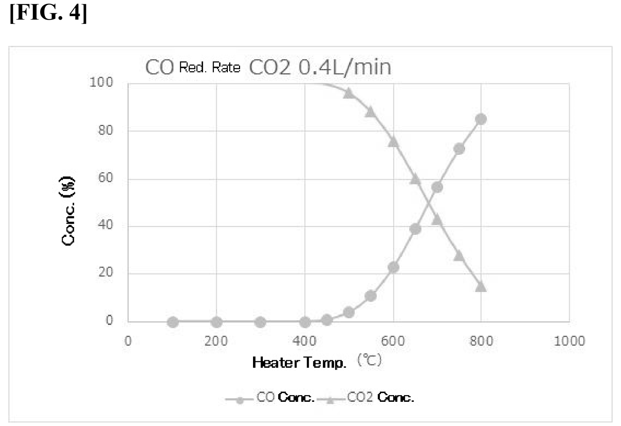

Subsequently, supplying CO₂ at room temperature at a flow rate of 0.4 L/min instead of stopping the supply of nitrogen, the temperature of the furnace was heated up to around the certain heater temperature, measuring CO level and CO₂ level until the levels reached the equilibrium. The relationship between the temperature in a furnace and the equilibrium concentration of CO and CO₂ was shown in FIG.4. The concentrations of CO and CO₂ were measured by sampling produced gas from discharge pipe open to the atmosphere installed at the bottom of the outer peripheral of the furnace by use of NDIR gas sensor (gas flow rate: 0.5 L/min, manufactured by AWIT).

As can be seen from FIG. 4, as heater temperature gets elevated from 400°C to 800°C, CO gets increased from 0% to 85%, whereas CO₂ was decreased from 100% to 15%. Given the following formula:

C + CO₂ ↔ 2CO

the equilibrium concentration at 800°C is close to the theoretical concentration of 87% (RTlnK = -ΔG°, ΔG° = -17.5 kJ·mol⁻¹@800°C, data reference: Central Research Institute of Electric Power Industry Report M19002 Basic characteristics of tubular type molten carbonate direct carbon fuel cells), suggesting that produced carbon attached to the catalyst was reacted with CO₂ to produce CO where Boudouard reaction took place.

### (Experimental Example 3 - Time course of Boudouard reaction)

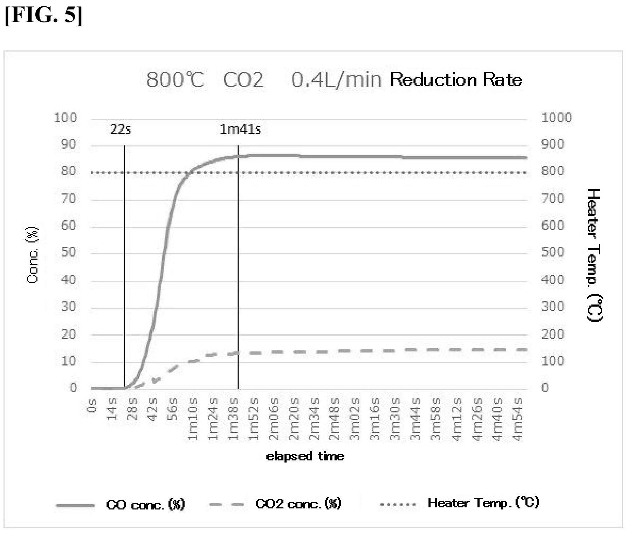

In Experimental Example 1, the supply of methane was stopped and nitrogen gas was filled at a flow rate of 0.4 L/min for 30 minutes at room temperature in reaction furnace where metal catalyst plate being installed with produced carbon being attached to the metal catalyst plate to purge methane and hydrogen remained in the furnace. Subsequently, once the supply of nitrogen gas was stopped, CO₂ at the room temperature was supplied into the furnace at a flow rate of 0.4 L/min. Given the timing of switching from nitrogen gas to CO₂ be 0 second, the time course of CO and CO₂ concentration levels were observed on the second timescale. The result is shown in FIG. 5.

As can be seen from FIG. 5, the reduction started at 22 seconds after switching from nitrogen to CO₂, and at 1 minute 41 seconds, reached a steady CO concentration of 83%. This suggests that one might go through the decrease of temperature at the start of introducing CO₂, shortly afterwards, the temperature in the furnace was returned to around 800°C, thereby drastically proceeding CO reduction, and one might not see the increase of the concentration of CO₂ being supplied but see the conversion into CO. At this stage, it was believed that there might have remained produced carbon sufficient to reduce CO₂ in the furnace.

It should be noted that the embodiments for carrying out the present invention are not at all limited to the above embodiments, nor all the elements explained in the above embodiments are the essential elements for the present invention. The present invention may go through various modifications insofar as it falls within the technical scope and to the extent that falls within the technical idea.

In an alternative embodiment of CO₂ cyclic usage system 1, there may be a CO₂ storage tank between dehydration device 7 and SDRM reaction module 11. The CO₂ storage tank may store when methane demand is high and discharge when methane demand is low, thereby adjusting the amount of CO to be supplied to methanation module 15 so as not to exceed or fall below 33% the production amount of hydrogen.

In an alternative embodiment of CO₂ cyclic usage system 1, supposing that air is used as an oxidizing agent used for combustion in methane oxidation module 3, there may be an apparatus to separate CO₂ from CO₂, nitrogen molecule and nitrogen oxides etc. that are contained in combustion gas on the latter stage of methane oxidation module 3.

In an alternative embodiment of SDRM reaction module 11, there may be a pipe between purification apparatus 36 and compressor 40a for returning a raw material gas of methane separated at purification apparatus 36 to reaction furnace 33 or methane inlet 42, and there may be a pipe between purification apparatus 38 and compressor 40b for returning a raw material gas of CO₂ separated at purification apparatus 38 to reaction furnace 33 or CO₂ inlet 44.

In an alternative embodiment of SDRM reaction module 11, there may be a compressor in the upstream of purification apparatuses 36, 38 (a side close to gas outlet).

In an alternative embodiment of SDRM reaction module 11, there may be a main valve at a position closer than third valve or fourth valve to gas outlet of reaction furnace capable of discharging a gas remained in said reaction furnace into the atmosphere or a main valve capable of releasing said remained gas into a path different from each path of produced gas. The purging operation at the main valve using raw material gas may improve the purity of produced gas controlled by third valve and fourth valve downstream of the main valve.

In an alternative embodiment of SDRM reaction module 11, there may be one pair of gas inlet and gas outlet. A path heading from the gas inlet to the supply source of each raw material gas or a path heading from the gas outlet to third valve and fourth valve may be unified partly. At each branched point, there may be one three-way valve on an inlet side in place of first valve and second valve, while there may be one three-way valve on an outlet side in place of third valve and fourth valve.

In an alternative embodiment of SDRM reaction module 11, there may be a plurality of apparatuses described in FIG. 7 (two apparatuses of Apparatus A and Apparatus B), wherein the reactions of formula (i) or formula (j) were done in apparatus A and the reaction of formula (k) was done in apparatus B for a while, and when solid carbon accumulates in apparatus A, the reaction of formula (k) was done in apparatus A and the reaction of formula (i) or formula (j) were done in apparatus B. Such apparatus or method allows us to produce carbon monoxide and hydrogen simultaneously and continuously. At least three apparatuses may prevent the downtime even if one could encounter any apparatus/pipe trouble.

### [Industrial applicability]

A method of producing carbon monoxide or carbon monoxide and hydrogen of the present invention may have less concern about producing water in production process of carbon monoxide or synthesis gas, and may be suitable for the production of useful chemical products with a raw material of CO₂, and thus have a great industrial applicability. Particularly, it is preferably applicable to the synthesis of oxygen-containing compounds such as acetic acid (CH₃COOH), acrylic acid (C₃H₄O₂), benzoic acid (C₇H₆O₂) and polycarbonate (C₁₆H₁₄O₃)ₙ. The present invention is also preferably applicable to onsite (i.e. at the place of use) production and supply of highly toxic carbon monoxide, as well as the utilization of a large amount of excess CO₂ from CCS.

A method of producing methane or propane by the cyclic usage of CO₂ according to the present invention may not require hydrogen purchasing even when consumers who collect and cyclically use CO₂ generated by using domestic/foreign natural gas, city gas and biogas implement the method onsite (e.g. in a plant site of each manufacturer, or a plant site of LNG thermal power plant that generate CO₂) or when energy suppliers implement the method offsite (e.g. in a plant site of city gas), and reduce the cost for the increase of methane supply by monetizing product carbon, thereby achieving lower running cost. Particularly it is advantageous for energy suppliers to avoid the decrease or loss of the sales amount of city gas, which was unavoidable in the conventional methanation system using hydrogen, and furthermore may increase the sales amount of city gas by 1.5 to 3 times. Therefore, the present invention may have great industrial applicability. Furthermore, the present method may have great applicability due to the availability of propane tank if consumers who collect and cyclically use CO₂ were cement plants, because these plants are often located suburban area without city gas pipeline.

### [Explanation of Symbols]

1, 101, 121, 131, 141, 151, 161 CO₂ cyclic usage system
3, 103 Methane oxidation module
53 Propane oxidation module
7, 17, 107, 117 Dehydration device
8, 108 Pipeline
11, 21, 111 SDRM reaction module
31 Propane pyrolysis module
41 Methane pyrolysis module
15, 65, 115 Methanation module
55 Propanation module
18 Propane tank
24 Catalyst hanger
25 Thermocouple
33, 23 Reaction furnace
54 Desulfation device
35 Heating apparatus
36 H₂ purification apparatus
37, 27 Structural catalyst
38 CO purification apparatus
39 First valve
40a, 40b Compressor
41 Second valve
42 Methane inlet
43 Third valve
44 CO₂ inlet
45 Fourth valve
46 Hydrogen gas outlet
47 CH₄ supply source
48 CO gas outlet
49 CO₂ supply source
102 Site of city gas supplier
104 Site of city gas consumer
106 Transportation means
109 CO₂ compressor/liquefier
110 CO₂ storage facilities

## Claims

1. A method of producing carbon monoxide or carbon monoxide and molecular hydrogen, comprising the steps of proceeding the reactions represented by the following formulae (1) and (2) in a sequential order within the identical container substantially in the anhydrous condition, wherein the reaction of the following formula (1) is conducted using a structural catalyst having a catalytic activity of methane pyrolysis reaction:
CH₄ → C + 2H₂ (1)
C + CO₂ → 2CO (2)

2. The method of Claim 1, wherein said carbon dioxide (CO₂) is CO₂ stored by the carbon dioxide collection/storage (Carbon Capture Storage; CCS) technique.

3. The method of Claim 1, wherein said reaction of formula (2) is conducted in the presence of carbon produced in said reaction of formula (1).

4. A method of producing a synthesis gas or a chemical product using the same, comprising the step of mixing carbon monoxide and molecular hydrogen obtained by said method of Claim 1 in a certain ratio by the molar flow rate adjustment.

5. The method according to Claim 4, further comprising the step of adding molecular hydrogen from renewable energy to adjust a H₂/CO ratio.

6. A reaction apparatus comprising: a reaction furnace containing a structural catalyst having a catalytic action of methane pyrolysis reaction; and a CO detector on a flow path communicated with a gas outlet of said reaction furnace.

7. The reaction apparatus according to Claim 6, wherein said reaction furnace comprises a gas inlet and said gas outlet, and further comprises a three-way valve on an inlet side to switch a supply path to said gas inlet and a three-way valve on an outlet side to switch a supply path from said gas outlet.

8. The reaction apparatus according to Claim 6, further comprising an outlet valve to control the discharge of molecular hydrogen and/or CO from said reaction furnace, and a main valve capable of discharging a gas remained in said reaction furnace into the atmosphere or a main valve capable of releasing said remained gas into a flow path different from either hydrogen flow path or CO flow path.

9. A method of producing methane or propane by cyclic usage of CO₂ produced following oxidation reaction of hydrocarbons, comprising:
a step (1) of producing molecular hydrogen and/or CO₂ following oxidation reaction of hydrocarbons;
a step (2) of producing C and molecular hydrogen by pyrolysis of hydrocarbons;
a step (3) of producing CO by reacting CO₂ produced in the step (1) with carbon produced in the step (2);
and
a step (4) of producing methane or propane by reacting CO produced in the step (3) with molecular hydrogen produced in the step (1) or step (2),
wherein the reactions of said step (2) and step (3) are conducted in a sequential order substantially in the anhydrous condition within the identical container containing a structural catalyst having a catalytic activity of methane pyrolysis reaction.

10. The method of producing methane or propane according to Claim 9 further satisfying the following condition (I) or (II):
(I) further comprising a step (5A) of using methane or propane produced in the step (4) as hydrocarbons for the step (1); or
(II) further comprising a step (5B) of using an unreacted material of hydrocarbons used in the step (2) for the step (1).

11. The method of producing methane or propane according to Claim 9, wherein said step (2) is a step of producing carbon and molecular hydrogen by the pyrolysis of methane 1.5 times or more or 5/6 time or more the amount of CO₂ to be cyclically used in molar ratio.

12. The method of producing methane or propane according to Claim 9, wherein an amount of methane for pyrolysis in said step (2) is 3 times or more the amount of CO₂ to be cyclically used in molar ratio,
and a remaining amount of methane produced in said step (4) is used for the step (2).

13. The method of Claim 9, wherein the reaction of said step (3) is conducted in the presence of carbon produced in the reaction of said step (2).
